# EUROPEAN PATENT APPLICATION

(11) **EP 4 212 518 A1**
(43) Date of publication of application: **19.07.2023**
(21) Application number: 22151959.8
(22) Date of filing: 18.01.2022
(51) Int. Cl.: C07D 307/12, C08G 63/672, C08L 67/02

(54) **METHODS FOR THE SYNTHESIS OF DI(HYDROXYMETHYL)TETRAHYDROFURAN AND ITS APPLICATION IN POLYESTERS AND POLYURETHANES**

(71) Applicant: Henkel AG & Co. KGaA, 40589 Düsseldorf (DE)
(72) Inventor: SPIEGELBERG, Brian, 40627 Düsseldorf (DE); BRANDT, Adrian, 45219 Essen (DE); BECK, Horst, 41470 Neuss (DE); KUX, Alexander, 40789 Monheim (DE); TIN, Sergey, 18055 Rostock (DE); DE VRIES, Johannes Gerardus, 6228GZ Maastricht (NL)
(74) Representative: Viering, Jentschura & Partner mbB Patent- und Rechtsanwälte

(57) **Abstract**

The present invention relates to the synthesis of di(hydroxymethyl)tetrahydrofuran (DHMTHF) and the preparation of trans-enriched mixtures of DHMTHF. The invention further refers to polyester polyols which are obtained by reaction of cis/trans DHMTHF mixtures with a dicarboxylic acid. The invention further relates to polyurethanes and compositions containing a polyester polyol comprising DHMTHF, in particular to such polyurethane-containing (adhesive) compositions.

## Description

The present invention relates to the synthesis of di(hydroxymethyl)tetrahydrofuran (DHMTHF) and the preparation of trans-enriched mixtures of DHMTHF. The invention further refers to polyester polyols which are obtained by reaction of cis/trans DHMTHF mixtures with a dicarboxylic acid. The invention further relates to polyurethanes and compositions containing a polyester polyol comprising DHMTHF, in particular to such polyurethane-containing (adhesive) compositions.

The demand for novel polymers in the field of adhesive technologies is constantly growing due to the diverse requirements for a wide range of applications that adhesives need to fulfill. To this regard, substantial progress has been made during the last decades through the development of new synthetic polymers. However, the latter are mainly derived from fossil sources and they are consequently characterized by a low level of sustainability. On the contrary, the use of renewable feedstock materials could offer a reduction of the carbon footprint of the final products, and at the same time it could provide access to new chemical structures, not available from traditional petro-based sources.

Among the different classes of adhesives, hot melts are characterized by a current growth rate 1.5-2 times higher than the ones registered for other types of adhesives. Typical components for reactive polyurethane hot melt adhesives are industrially produced through the reaction of polyisocyanates with either polyether polyols or polyester polyols. Notably, bio-based polyesters polyols, polyether polyols, and polyurethanes have been reported to an increasing extent both in industry and in academia. However, research regarding the influence of different stereoisomers of bio-based polyols on the properties of the derived polymers and materials is scarce.

The use of the different diastereomers of the bio-based diol di(hydroxymethyl) tetrahydrofuran (DHMTHF) for the synthesis of polyesters has been reported in the art (Moore & Kelly, Macromolecules 1978, 11, 568-573). However, in the reported methods, the authors obtained these polymers by using chloroform, a non-benign solvent, carboxylic acid chlorides as co-monomers, and in the presence of an excess of triethylamine. Furthermore, two weeks were required for the polymerization process and the products consisted of only low molecular weight materials. Finally, the *cis* and *trans* DHMTHF isomers were synthesized through a multi-step process including a reduction with sodium amalgam and stochiometric reactions with toxic and explosive diazomethane and lithium aluminum hydride, respectively.

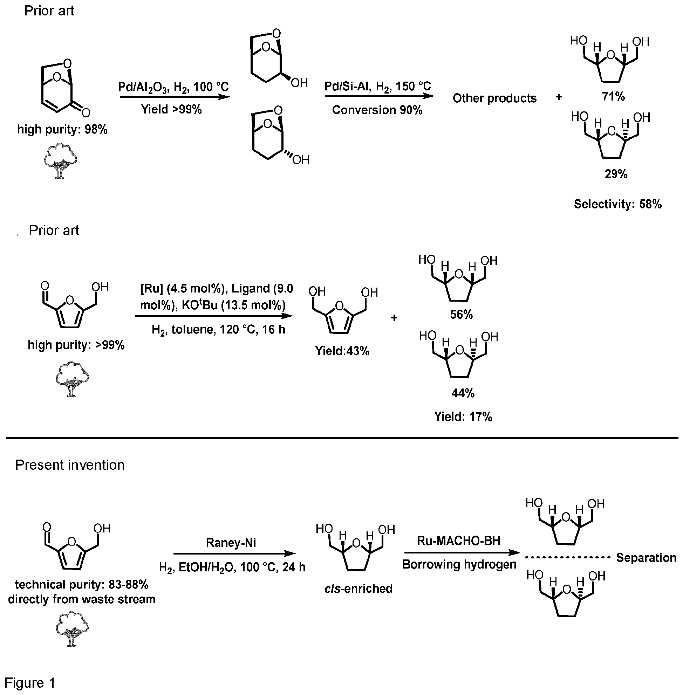

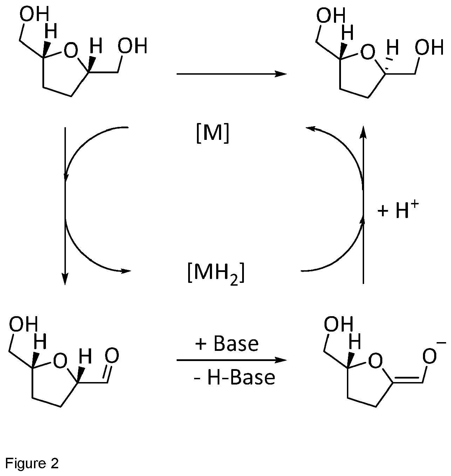

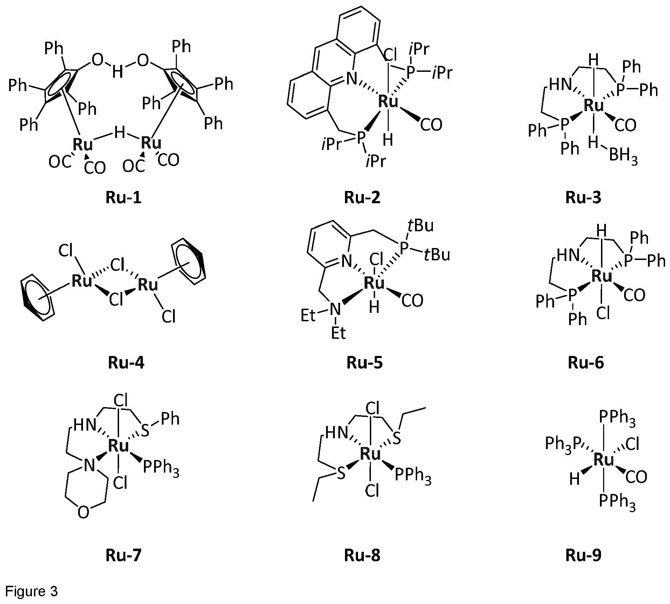

Recently, DHMTHF was used for the synthesis of copolyesters with 1,4-cyclohexanedimethanol and furandicarboxylic acid (FDCA) and it was revealed that by increasing the content of *cis*-DHMTHF in the synthesized polymers an increase of stiffness, storage modulus, and hydrophobicity of the latter can be obtained (Jin et al. ACS Sustainable Chem. Eng. 2021, 9, 39, 13287-13302). Notably, even if the authors used exclusively the *cis* isomer of DHMTHF, it can be assumed that the corresponding *trans* isomer would have led to different properties due to changes of the spatial shape and alignment in the resulting polymers. Although *cis*/*trans*-DHMTHF mixtures were disclosed for FDCA polyesters (WO 2017/091435 A1; WO 2017/091412 A1), an economically feasible way of synthesizing pure or *trans* enriched DHMTHF is not known so far. In fact, in these disclosures DHMTHF was only obtained with a moderate selectivity of 58% and a maximum *trans* content of 29% from the hydrogenation of levoglucosenone (LGO) in the presence of supported palladium catalysts at 150 °C (Figure 1). Additionally, a series of homogenous ruthenium precursors in combination with NHC or di-phosphorus containing ligands was reported for the hydrogenation of 5-HMF to DHMTHF which was obtained with a *trans* content up to 44% albeit with a very poor yield of 17% (Cadu et al. Green Chem. 2018, 20, 3386-3393). In this latter case, the authors used high catalyst loadings, and both reported procedures led to product mixtures from which the isolation of DHMTHF would be problematic. Beyond that, both LGO and 5-HMF were employed as high-purity, crystalline starting materials, which would certainly make DHMTHF far too expensive as a polymer building block.. The high cost of the latter is a consequence of the purification procedures needed to remove impurities like formic acid, levulinic acid and oligomers.

Herein, it was found that a low-purity 5-HMF aqueous solution, obtained as a side product of a hydrothermal carbonization process of sugars or lignocellulosic material (Thoma et al. ChemSusChem 2020, 13, 3544-3564), can be utilized to synthesize DHMTHF and its corresponding polymers.

According to current research, the introduction of linear molecules containing ether bonds causes favorable effects on polarity, hydrophilicity, degradability, and biocompatibility of polyesters; however, they usually have a significantly negative impact on strength and thermal properties. The present inventors hypothesized that, from the perspective of molecular structures, replacing the linear monomer containing ether bonds with cyclic monomers may be a route to overcome the problems. While, to date, several kinds of cyclic diols, such as 1,4- cyclohexanedimethanol (CHDM), 2,2,4,4-tetramethyl-1,3-cyclobutanediol (CBDO), and isosorbide (IS), have been reported to improve the properties of synthesized polyesters, from these named cyclic diols only isosorbide is currently available from biomass feedstock materials.

Although DHMTHF may be a promising novel bio-based monomer to allow the synthesis of new materials with attractive properties, to date no scalable method is known to synthesize trans enriched DHMTHF, which certainly causes a difference in the properties in the resulting polymers compared to the corresponding cis DHMTHF analogues. Further, the use of DHMTHF as main diol in polyester polyols which in turn are suitable for polyurethane adhesives that exhibit at the same time good tensile strength and high elongation is unknown. In order to benefit from the advantageous properties of DHMTHF derived polyester polyols it would therefore be desirable to be able to employ these in other systems as well.

It is therefore the object of the present invention to provide polyester polyols comprising a bio-based monomer which can be used in liquid systems at ambient temperature applications such as polyurethane adhesives, in particular two component polyurethane adhesives. Further, the latter need to meet the requirements of industrial use for instance to join materials with different coefficients of thermal expansion.

It was surprisingly found that the above objects are solved by the development of a new synthetic pathway to obtain DHMTHF with a high trans isomer content and further by its use for the synthesis of polyester polyols obtainable from reaction mixtures comprising a defined cis/trans DHMTHF mixture and a dicarboxylic acid, preferentially derived from biomass.

In a first aspect, the present invention is therefore directed to a method for the production of di(hydroxymethyl)tetrahydrofuran (DHMTHF) from 5-hydroxymethylfurfural (5-HMF), the method comprising hydrogenation of a solution of 5-HMF, such as an aqueous solution, in the presence of a heterogeneous catalyst, wherein the 5-HMF is crude 5-HMF.

"Aqueous solution", as used herein, refers to a solution with water as the main solvent, i.e. water constitutes at least 50 wt.-% or vol.-% of the total solvent used, for example at least 70, at least 80 or at least 90 wt.-% or vol.-%.

In various embodiments, the heterogeneous catalyst is a syn facial hydrogenation mode catalyst, preferably selected from Raney nickel, Ru/C (ruthenium on carbon), Pd/AI (palladium on alumina), and Pd/C (palladium on carbon), more preferably Raney nickel. Further suitable catalysts include, without limitation, Pd/Si (palladium on silica), Ru/AI (ruthenium on alumina), Raney cobalt, and Raney copper.

In various embodiments, the hydrogenation reaction is carried out in the presence of ethanol in an amount of at least 1.3 mL ethanol per 1 mmol 5-HMF, preferably 1.4 to 3.0 mL ethanol per 1 mmol 5-HMF.

In various embodiments, the hydrogenation reaction is carried out under a H₂ pressure of at least 10 or at least 20 bar, preferably at least 50 bar, more preferably 80 to 100 bar; and/or at a temperature of 80 to 120°C, preferably at about 100°C; and/or at a reaction time of at least 5 hours, preferably 5 to 60 hours.

In these methods, the 5-HMF used is crude 5-HMF. Such crude 5-HMF may be a side stream product in a hydrothermal carbonization process, for example of a sugar or lignocellulosic material. Generally, the crude 5-HMF may have a purity of less than 95%, for example less than 94, less than 93, less than 92, less than 91, less than 90, less than 89, less than 88, less than 87% (all by weight), relative to the total content of organics. In various embodiments, 5-HMF purity is in the range of 70 to 95%, such as 75 to 90 %, for example 80 to 90 %. "Purity", as used in this context, relates to the purity of the solid or organic fraction, i.e. without any potentially present aqueous phase/water. The crude 5-HMF may be provided in form of an aqueous solution that comprises, for example 15-25 wt.-% 5-HMF solid content.

The impurities contained in the crude 5-HMF may comprise those selected from formic acid, levulinic acid, oligomers of 5-HMF and combinations thereof, optionally in an amount of at least 1 wt.-%, at least 2wt.-%, at least 3wt.-%, at least 4, 5, 6, 7, 8, 9, 10, 11, 12, 13, 14, 15, 16, 17, 18, 19 or 20 wt.-% of the total weight of organics in the crude 5-HMF.

In various embodiments, these methods provide for cis enriched DHMTHF, i.e. the content of the cis diastereomer is higher than that of the trans diastereomer, preferably the molar ratio is greater than 1, more preferably greater than 1.5, greater than 2, greater than 3, 4, 5, 6, 7, 8, 9, 10, 11, 12, 13, 14, 15, 16, 17, 18, 19 or 20. In various embodiments, the cis to trans ratio is at least 80:20, preferably at least 85:15, more preferably at least 90:10, for example about 92:8.

In another aspect, the present invention is directed to a method for the enrichment of trans-DHMTHF from a mixture of cis-/trans-DHMTHF, the method comprising reacting a mixture of cis-/trans-DHMTHF at elevated temperatures in the presence of a suitable metal catalyst for a metal catalyzed borrowing hydrogen reaction, a base and a suitable solvent.

"Elevated temperature", as used herein, relates to a temperature above ambient, i.e. a temperature of 30°C or higher, such as at least 40, at least 50, at least 60 or at least 70°C.

In such methods, the mixture of cis-/trans-DHMTHF used as a starting material has a cis to trans ratio of at least 1, preferably at least 2, more preferably 3, 4, 5, 6, 7, 8, 9, 10, 11, 12, 13, 14, 15, 16, 17, 18, 19 or 20. or 1-20, 2-20 or 3-20. In various embodiments, it may be the DHMTHF obtainable according to the hydrogenation methods described herein.

The trans enriched DHMTHF obtainable according to the methods described herein refers to mixtures of cis-/trans-DHMTHF in which the content of the trans diastereomer is at least 30 mol-%, preferably at least 35 or at least 40 mol-% relative to the total amount of cis and trans diastereomers. These are considered trans enriched relative to the cis/trans mixtures used as a starting material which typically contain an excess of the cis diastereomer. Such an enrichment typically means that the amount of trans diastereomer relative to the starting material is increased by at least 5 mol-%, preferably at least 10 mol-%, preferably 20 mol.-% and more. For example, using a cis/trans DHMTHF mixture with a cis/trans ratio of 90:10, the enrichment methods described herein may provide for trans enriched mixtures that have a trans content of at least 30 mol-%, i.e. the cis trans ratio is 70:30 or lower, such as 60:40.

In various embodiments, the catalyst for a metal catalyzed borrowing hydrogen reaction is a metal complex catalyst, such as a ruthenium complex catalyst, a cobalt complex catalyst, a manganese complex catalyst, or an iron complex catalyst.

In various embodiments, it is a ruthenium complex catalyst, preferably a ruthenium complex bearing tridentate pincer ligands. Such a catalyst may be selected from, without limitation,
Carbonylchlorohydrido[bis(2-di-cyclohexylphosphinoethyl)amine]ruthenium(II);
Carbonylchlorohydrido[bis(2-di-i-propylphosphinoethyl)amine]ruthenium(II),
Carbonylchlorohydrido[bis(2-di-t-butylphosphinoethyl)amine]ruthenium(II),
Dichloro[rel-[N(S)]-N-[2-[(R)-phenylthio-κS]ethyl]-4-morpholineethanamine-κNN4,κN4](triphenylphosphine)ruthenium(II),
Dichloro[rel-[N(R)]-N-[2-[(R)-(phenylmethyl)thio-κS]ethyl]-4-morpholineethanamine-κNN4,κN4](triphenylphosphine)ruthenium(II),
Dichloro[N-[2-(phenylthio-κS)ethyl]-[4-morpholineethanamine-κNN1,κN1](tricyclohexylphosphine)ruthenium(II),
Dichloro[rel-[N(S)]-N-[2-[(R)-phenylthio-κS]ethyl]-[1-pyrrolidineethanamine-κNN1,κN1](triphenylphosphine)ruthenium(II),
Dichloro[N1,N1-dimethyl-N2-[2-(phenylthio-κS)ethyl]-1,2-ethanediamine-κN1,κN2](tricyclohexylphosphine)ruthenium(II),
Dichloro[N-[2-(diphenylphosphino-κP)ethyl]-2-(methylthio-κS)ethanamine-κN](triphenylphosphine) ruthenium,
Dichloro[rel-[N(S)]-N-[2-(diphenylphosphinyl-κO)ethyl]-2-[(R)-methylthio-κS]ethanamine-κN](triphenylphosphine) ruthenium,
Carbonylchlorohydrido[bis(2-(diphenylphosphinoethyl)amino]ruthenium(II),
Carbonylchlorohydrido[6-(di-t-butylphosphinomethyl)-2-(N,N-diethylaminomethyl)pyridine]ruthenium(II),
Carbonylchlorohydridotris(triphenylphosphine)ruthenium(II),
Carbonyl(dihydrido)tris(triphenylphosphine)ruthenium(II),
Carbonylhydrido[6-(di-t-butylphosphinomethylene)-2-(N,N-diethylaminomethyl)-1,6-dihydropyridine]ruthenium(II),
Carbonylhydrido(tetrahydroborato)[bis(2-diphenylphosphinoethyl)amino]ruthenium(II), Chlorocarbonylhydrido[4,5-bis-(di-i-propylphosphinomethyl)acridine]ruthenium(II),
Chlorohydridotris(triphenylphosphine)ruthenium(II),
1-Hydroxytetraphenylcyclopentadienyl(tetraphenyl-2,4-cyclopentadien-1-one)-µ-hydrotetracarbonyldiruthenium(II),
Dichloro(benzene)ruthenium(II) dimer,
Carbonyl(dihydrido)tris(triphenylphosphine)ruthenium(II),
Chlorohydridotris(triphenylphosphine)ruthenium(II), and
[Ru (1,1,1-tris(diphenylphosphinomethyl)ethane)TMM] (TMM = Trimethylenmethane).

In various embodiments, the ruthenium catalyst is selected from: 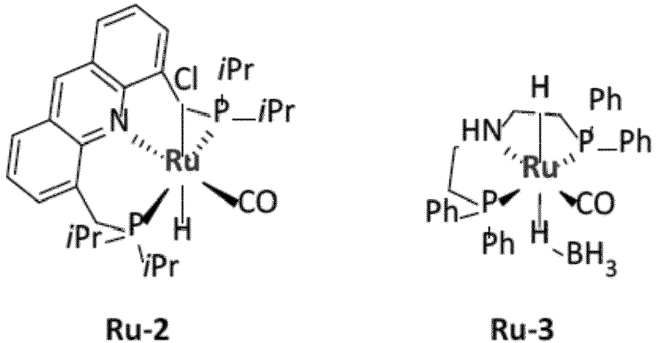 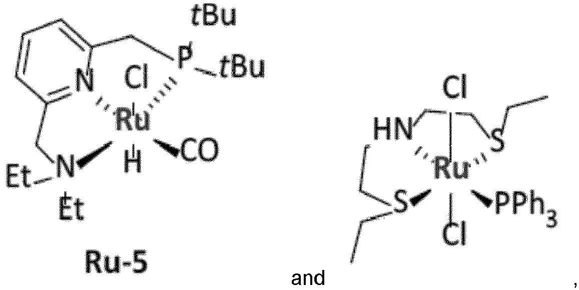 preferably Ru-2 and Ru-3, for example Ru-3.

Other suitable catalysts include, without limitation,
[N2,N4-Bis(diisopropylphosphino)-6-phenyl-1,3,5-triazine-2,4-diamine]-cobalt dichloride,
[N2,N4-Bis(diisopropylphosphino)-6-cyclopropylamino-1,3,5-triazine-2,4-diamine]-cobalt dichloride,
[N2,N4-Bis(diisopropylphosphino)-6-methyl-1,3,5-triazine-2,4-diamine]-manganese(dicarbonyl) bromide,
[N2,N4-Bis(diisopropylphosphino)-6-phenyl-1,3,5-triazine-2,4-diamine]-manganese dichloride;
[N2,N4-Bis(diisopropylphosphino)-6-cyclopropylamino-1,3,5-triazine-2,4-diamine]-manganese dichloride,
[N2,N4-Bis(diisopropylphosphino)-6-diethylamino-1,3,5-triazine-2,4-diamine]-manganese(dicarbonyl) bromide, and
Carbonylhydrido(tetrahydroborato)[bis(2-diphenylphosphinoethyl)amino]iron(II).

The catalyst may be used in amounts of the catalyst is used in amounts of 1 mol.-% or less, preferably about 0.5 mol.-% or less, for example about 0.5 mol%, 0.4 mol-%, 0.3 mol-%, 0.2 mol-% or 0.1 mol-%. It has been found that amounts of 0.1 mol-% still provide for good catalysis and may be even advantageous over significantly higher concentrations such as 1 mol-% and more.

In various embodiments, the reaction temperature is between 50 and 150°C, for example 80°C or more, preferably 80 to 120 °C;

In various embodiments, the base is a strong base. Such a strong base may be an alkoxide, in particular a metal alkoxide. Suitable bases, without limitation, include potassium tert butoxide, sodium tert butoxide, potassium tert pentoxide, sodium tert pentoxide, sodium methoxide, potassium methoxide, sodium ethoxide, and potassium ethoxide. In various embodiments, the base may be potassium tert butoxide (KOtBu) or potassium tert pentoxide (KOtPen). The base may be used in any suitable amounts, for example in amounts of at least 6, 7, 8, 9 or at least 10 mol.-%. It has been found that lower amounts may negatively affect the reaction while higher concentrations do not provide for additional benefits.

In various embodiments, the solvent is an organic solvent, preferably selected from, without limitation, heptane, THF, 1,4-dioxane, toluene, sulfolane, acetonitrile, 2-MeTHF, and mixtures thereof, for example toluene.

The method may be carried out under nitrogen or argon atmosphere. It may be advantageous that the reaction is not carried out under hydrogen atmosphere.

In various embodiments, the reaction time is 2 to 48 hours, preferably about 4 to 24 hours.

In another aspect, the invention relates to an alternative method for a method for the enrichment of trans-DHMTHF from a mixture of cis-/trans-DHMTHF, the method comprising
(a) acetylating the mixture of cis- and trans-DHMTHF, for example with acetic anhydride, optionally at elevated temperatures, to obtain a mixture of diacetyl esters of cis- and trans-DHMTHF;
(b) crystallizing the diacetyl ester of trans-DHMTHF from a solution of the mixture of diacetyl esters of cis- and trans-DHMTHF in a suitable solvent, at a temperature that allows preferential crystallization of trans DHMTHF, preferably a temperature of between -5 to -20°C, more preferably at about -15°C;
(c) isolating the crystallized diacetyl ester of trans-DHMTHF; and
(d) hydrolyzing the diacetyl ester of trans-DHMTHF under basic conditions to obtain enriched trans-DHMTHF.

Step (a) may be followed by a step of removing acetic acid, if such is formed in step (a).

In these methods, the mixture of cis-/trans-DHMTHF used as a starting material has a cis to trans ratio of 3 or less, preferably 2.5 or less, more preferably 2 or less, most preferably 1.5 or less, but typically higher than 1. In various embodiments, it may be the DHMTHF obtainable according to the hydrogenation methods described herein. In various other embodiments, the mixtures employed for these methods are those produced by the catalyzed trans enrichment method described above.

The definition for trans enriched DHMTHF provided above also applies to this method.

In various embodiments, the diacetyl esters remaining after removal of the acetic acid in step (b) are dissolved in a suitable solvent to allow crystallization/precipitation of the trans diastereomer. The solvent may be, without limitation, diethyl ether, THF, 2-MeTHF, 1,4-dioxane, methyl tertbutyl ether, cyclopentyl methyl ether, tertamyl ethyl ether, acetonitrile and ethyl acetate. The enrichment of the trans diastereomer is based on the finding that the trans isomer crystallizes at higher temperatures than the cis diastereomer. In the described method and in the described solvent, the temperature at which the trans diastereomer of diacetyl DHMTHF precipitates from the solution is in the range of about -5 to about -20°C, preferably at about -15°C. It has been found that at lower temperatures, for example at about - 30°C, both diastereomers precipitate from the solution. It is understood that these temperature ranges may differ in other solvent systems and under different conditions. In various embodiments, solvent systems are used in which the temperature at which one of the two diastereomers crystallizes is at least 5°C higher than that at which the other crystallizes, preferably at least 10 °C higher. Generally, all temperatures used for crystallization may be lower than 5°C or lower than 0°C.

"About", as used herein in relation with numerical values, refers to the reference value ±10%, preferably ±5%.

The isolation of the trans enriched diacetyl DHMTHF may be carried out by centrifugation and decantation or filtration and subsequent drying. The crystalline precipitate may be washed before drying, for example with diethyl ether for multiple times to remove impurities. The drying may then be carried out in vacuo, i.e. under reduced pressure. The thus obtained trans enriched diacetyl DHMTHF may have a cis:trans ratio of 30:70 or lower, preferably 20:80 or lower, for example about 15:85.

The trans enriched diacetyl DHMTHF may then be hydrolyzed to yield the trans enriched DHMTHF. For this reaction the crystalline material may be dissolved in a suitable solvent, such as ethanol, and then a base may be added, for example NaOH, e.g. as an aqueous solution. Suitable reaction conditions are known to those skilled in the art and may entail adding the base slowly over an extended period of time and extensive stirring for extended periods of time, such as 48 hours. The resulting water may be removed under reduced pressure and the residue filtered and eluted with a suitable solvent, such as ethyl acetate. All volatiles may then again be removed under reduced pressure to obtain the trans enriched DHMTHF as a colorless liquid.

The thus obtained trans enriched DHMTHF may retain the previous cis/trans ratio and may have a cis:trans ratio of 30:70 or lower, preferably 20:80 or lower or even 10:90 and lower, for example about 15:85.

In these acetylation methods, the supernatant remaining in step (d) that contains the cis enriched diacetyl DHMTHF may be subjected to a similar hydrolyzation process as the trans enriched diacetyl DHMTHF to obtain cis enriched DHMTHF, which may have a cis/trans ratio of 60:40 or higher, for example 70:30 or higher. This cis enriched DHMTHF may then be subjected to the borrowing hydrogen method to provide for trans enriched DHMTHF which can then be again subjected to the acetylation method for further trans enrichment. If this sequence of steps is repeated multiple times, a nearly stereoselective formation of trans-DHMTHF can be achieved. Such an approach involves both trans enrichment methods described herein.

The present invention also relates to the thus obtained trans enriched DHMTHF mixtures, in particular cis/trans DHMTHF mixtures that have a molar ratio of cis to trans diastereomer of 70:30 or less, preferably 70:30 to 30:70, for example 60:40 to 40:60.

Another aspect of the invention relates to a polyester polyol obtainable by reacting a diastereomeric mixture of cis-/trans-DHMTHF with a suitable diacid. The cis/trans mixtures of DHMTHF may be those obtainable to the methods described herein above. In various embodiments, the diastereomeric mixture of cis-/trans-DHMTHF has a cis- to trans-DHMTHF ratio of about 70:30 or less, for example of about 30:70 to about 70:30, preferably about 40:60 to about 60:40. Such mixtures are also referred to herein as trans enriched cis/trans DHMTHF mixtures.

In various embodiments, the diacid is at least one dicarboxylic acid. The dicarboxylic acid may be an aliphatic or aromatic dicarboxylic acid and may comprise 4 to 30 carbon atoms. If it is an aliphatic dicarboxylic acid it can be a saturated or unsaturated aliphatic dicarboxylic acid. In various embodiments, it is an aliphatic dicarboxylic acid, for example an aliphatic dicarboxylic acid having a carbon chain of at least 2 carbon atoms, which may be saturated or unsaturated. In various embodiments, it may be a saturated aliphatic C₄- to C₂₄-dicarboxylic acid, such as 1,6-hexanedioic acid, 1,7-heptanedioic acid, 1,8-octanedioic acid, 1,9-nonanedioic acid, 1,10-decanedioic acid, 1,11-undecanedioic acid, 1,12-dodecanedioic acid, 1,13-tridecanedioic acid, 1,14-tetradecanedioic acid, 1,15-pentadecanedioic acid, 1,16-hexadecanedioic acid, 1,17-heptadecanedioic acid, 1,18-octadecanedioic acid, 1,19-nonadecanedioic acid, 1,20-eicosanedioic acid, 1,21-heneicosanedioic acid, 1,22-docosanedioic acid, 1,23-tricosanedioic acid, and 1,24-tetracosanedioic acid as well as anhydrides, halides (chlorides) and ester derived from said acids. In other embodiments, the acids used include, without limitation, succinic acid, adipic acid, sebacic acid, azelaic acid, isophthalic acid, orthophthalic acid, terephthalic acid, furan dicarboxylic acid, itaconic acid as well as anhydrides, halides (chlorides) and ester derived from said acids. It is understood that mixtures and salts of all the above-listed acids as well as their derivatives, such as esters, halides and anhydrides, may also be used.

The expression "carbon chain" used in connection to the dicarboxylic acid refers to the linear carbon chain separating the two carboxylic acid groups, the chain being terminated by a carboxylic acid group on each end with the carbon of the carboxylic acid group being the start and end point, respectively, for determining the number of carbon atoms in the carbon chain.

In various embodiments, the polymerization reaction is carried out using a two-stage melt condensation process comprising a precondensation step at ambient pressure (i.e. about 1 bar) with a gradual temperature increase of from about 120 to about 220°C and a subsequent condensation step under reduced pressure, optionally in the presence of a suitable catalyst, preferably a polycondensation catalyst, such as a metal alkoxide, more preferably a titanium alkoxide catalyst, even more preferably titanium(IV)isopropoxide.

Examples of suitable polycondensation metal catalyst include, without limitation, aluminium alkoxides, titanium alkoxides, magnesium alkoxides and zirconium alkoxides, tin compounds, more particularly organotin carboxylates, such as dibutyltin dilaurate, dibutyltin diacetate, dibutyltin bis-(2-ethylhexanoate) or other organotin compounds, such as dibutyltin oxide, dibutyltin dimethoxide, dibutyltin dibromide, dibutyltin dichloride, ditert.butyltin dichloride, dimethyltin dibromide, dimethyltin dichloride, diphenyltin dichloride or tin octoate, iron acetate, iron benzoate, iron naphthenates; iron acetyl acetonates, manganese acetate, manganese naphthenate and manganese acetyl acetonate.

The molecular weight of the components comprised in the reaction mixture is determined according to standard procedure, for example by GPC or end group titration (OH value determination).

In a still further aspect, the present invention relates to a polyester polyol comprising monomeric units of formula (la) and (lb) 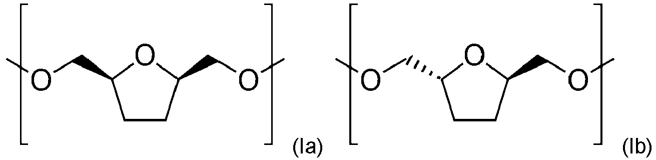 in a molar ratio of (la) to (lb) of 70:30 or less, preferably 70:30 to 30:70. In some embodiments, the monomeric units of formulae (la) and (lb) constitute at least 5 mol-%, at least 10, 15 ,20, 25, 30, 35, 40, 45, or at least 50 mol-% or at least 60, 70, 80, 90 or at least 95 mol-% of the total polyol units in said polyester polyol. In various embodiments, the ratio of units of formula (la) to units of formula (lb) is about 30:70 to about 70:30, preferably about 40:60 to about 60:40. In various embodiments, the polyester polyol comprises no polyol units other than those of formulae (la) and (lb) or these other polyols are present in amounts of 10 mol-% of all polyol units or less. In addition to these polyol-derived monomeric units, the polyester polyol also comprises diacid-derived monomeric units that connect the polyol-derived units. These acid units may be derived from the diacids disclosed above. Said polyester polyols can be produced according to the methods described herein.

It was further surprisingly found that some of the inventive polyester polyols are semi-crystalline while at the same time having a low melting point, making them especially suitable for temperature-sensitive applications. In one embodiment, the inventive polyester polyol has a (semi)-crystalline morphology at ambient temperatures and below. Ambient temperature as used in the present invention refers to a temperature of 23 to 25 °C at a pressure of 1000 to 1020 hPa.

A polyester polyol exhibiting a semi-crystalline morphology as described herein refers to a polyester polyol wherein the polymer chains are at least partially aligned. The morphology of the polyester polyol may, for example, be determined by DSC, wherein the crystallinity is usually expressed by defined melting and crystallization peaks in the diagram. In contrast thereto, amorphous materials are characterized by the absence of defined peaks in the DSC diagram.

Different processes for the production of (semi)-crystalline polyester polyols are known to the person skilled in the art. However, said crystalline polyester polyols usually have a melting or softening point well above room temperature. It was surprisingly found that the melting point of the inventive polyester polyol may be adapted by employing suitable amounts of trans-DHMTHF.

The melting point of the inventive polyester polyol may also be adjusted according to need by selecting suitable dicarboxylic acids.

In an especially preferred embodiment, the DHMTHF and dicarboxylic acids from which the inventive polyester polyol is derived from are in turn derived from renewable sources. Generally, such compounds obtained from renewable sources are referred to as "bio-based" compounds, in contrast to the common petrol-based compounds.

Some of the inventive polyester polyol is especially distinguished by its (semi)crystalline morphology while at the same time exhibiting a low melting point. In contrast to crystalline polyester polyols according to the state of the art which usually show high melting points above 80 °C, these polyester polyols were found to have melting points in low-temperature regions below 60 °C, allowing for more flexible applications in a number of technical fields. In one embodiment, the inventive polyester polyol has a melting point of -10 to 50 °C, preferably -5 to 30 °C, determined by DSC with a heating rate of 10 K/min.

In order to adjust the properties of the inventive polyester polyol, other components may be comprised in the reaction mixture in addition to the DHMTHF mixture and the at least one dicarboxylic acid. The reaction mixture can therefore comprise additional diols. Examples of such polyols include monoethylene glycol, 1,2-propanediol, 1,3-propanediol, 1,3-butanediol (including R-form, S-form and racemates), 1,4-butanediol, 1,4-pentanediol, 3-methylpentane-1,5-diol, neopentyl glycol (2,2-dimethyl-1,3-propanediol), 1,5-pentanediol, 1,6-hexanediol, 1,8-otaneglycol, cyclohexanedimethanol, 2-methylpropane-1,3-diol, diethyleneglycol, triethyleneglycol, tetraethyleneglycol, polyethyleneglycol, dipropyleneglycol, polypropyleneglycol, polypropyleneglycol, dibutyleneglycol and polybutyleneglycol.

It may however be preferred that such diols do not constitute more than 50 mol-% of the total amounts of polyols used, preferably about 30 mol-% or less or 20 mol-% or less or 10 mol-% or less.

In accordance with the later application, the reactivity of the inventive polyester polyol may also be adjusted according to need. In various embodiments, the inventive polyester polyol therefore has a hydroxyl value (OH value) of 5 to 250 mg KOH/g, 5 to 150, 10 to 100 mg KOH/g, or 20 to 50 mg KOH/g, or 25 to 34 mg KOH/g.

The hydroxyl value is a measure of the content of free hydroxyl groups in a chemical substance, usually expressed in units of the mass of potassium hydroxide (KOH) in milligrams equivalent to the hydroxyl content of one gram of the chemical substance. The analytical method used to determine hydroxyl value traditionally involves acetylation of the free hydroxyl groups of the substance with acetic anhydride in pyridine solvent. The hydroxyl value can be determined according to DIN 53240.

The hydroxyl value of the inventive polyester polyol can, for example, be adjusted by the ratio of diol to dicarboxylic acid in the reaction mixture. In a preferred embodiment, the molar ratio of the at least one diol to the at least one dicarboxylic acid in the reaction mixture is 1 .5:1 to 1 :1 , preferably 1 .2:1 to 1 :1.

If the polyester polyol is -OH terminated, the acid number is up to 20 mg/g, preferably up to 10, such as 0.5 to 10 or 1-3 mg/g.

In various embodiments, the polyester polyol may also be acid-terminated. In such embodiments, the acid number may be 5 to 250 and preferably 10 to 150 mg/g.

In a preferred embodiment, the inventive polyester polyol has an average molecular weight Mn of 1000 to 25000 g/mol, preferably 2000 to 10000 g/mol, determined by GPC. The molecular weight of the inventive polyester polyol can, in particular, be determined by GPC using THF as eluent.

The present invention, in another aspect, also relates to a method for the production of a polyester polyol as described above, the method comprising reacting a diastereomeric mixture of cis-/trans-DHMTHF with a suitable diacid. All embodiments disclosed above in relation to the polyester polyol also apply to these methods.

Some of the inventive polyester polyols are especially suitable for adhesive applications, in particular low melting systems. The presence of these polyester polyol in the adhesives allows benefitting from the advantageous properties of crystalline polyester polyols without the need of having to melt the adhesive at high temperatures.

A further aspect of the present invention is a composition, for example an adhesive or coating composition, comprising the DHMTHF mixtures or polyester polyols according to the invention. In these compositions the molar ratio of cis to trans DHMTHF, either as monomers or as monomeric units in a polymer, may be 70:30 or less, for example 70:30 to 30:70 or 60:40 to 40:60. Also encompassed is the use of the DHMTHF mixtures and polyester polyols described herein in adhesive or coating compositions.

The inventive polyester polyol can be applied in a number of adhesive systems. Preferably, the adhesive system is a polyurethane adhesive, in particular a two-component polyurethane adhesive (2K system) or a one-component polyurethane adhesive (1 K system). In various embodiments, the polyester polyol is thus used in polyurethane adhesives and other PU applications, such as foams, thermoplastic PU (TPU), PU dispersions (PUDs) and all other PU systems. It is further useful in other applications where polyesters are used, such as reactive prepolymers for acrylics and as tougheners in epoxies.

Accordingly, the present invention also relates, in a particular aspect, to the use of the mixture of cis/trans DHMTHF derived polyester polyols according to the invention as a constituent of a thermoplastic material or of an adhesive and/or sealant. While the mixture of polyester polyols according to the invention can be used both in thermoplastics as deforming and extrusion means or also as melting means for physically bonding adhesives and/or sealants, the polymeric material according to the invention can be used both in thermoplastics and as tackifying agent in adhesives , preferably in hot melt adhesives , as well as in cross-linking reactive adhesives, since the polymeric material according to the invention allows a low application temperature and a high elasticity and mechanical stability of the adhesive bond.

In one aspect, the invention is thus also directed to polyurethanes that are obtainable by reacting a polyester polyol as described herein or obtainable according to the methods of the invention with a polyisocyanate. Also encompassed are methods for the synthesis/production of such a polyurethane, comprising reacting a polyester polyol according to the invention or obtainable according to the methods of the invention with a polyisocyanate.

In addition to the polyester polyol of the invention, additional polyols may used, in particular those that are standardly used for the production of polyurethanes. It may however be preferred that the polyester polyols of the present invention constitute at least 5 mol.-%, at least 10 mol-%, at least 15 mol-%, at least 20, at least 25, at least 30, at least 35, at least 40, at least 45, at least 50 mol.-%, or at least 70 mol.-% or at least 80 or 90 or 95 mol-% of the total amount of polyols used.

Suitable additional polyols include, without limitation, polyhydroxy ethers (substituted or unsubstituted polyalkylene ether glycols or polyhydroxy polyalkylene ethers), polyhydroxy polyesters, the ethylene or propylene oxide adducts of polyols and the monosubstituted esters of glycerol, and "polymer polyols" (i.e., graft polyols containing a proportion of a vinyl monomer, polymerized in situ) as well as mixtures thereof. While such compounds are commercially available, methods for synthesizing such compounds are well known in the art. In various embodiments of the present invention, the polyols are selected from further polyester polyols, polyether polyol, and combinations thereof.

Suitable polyether polyols include linear and/or branched polyethers having plural numbers of ether bonds and at least two hydroxyl groups, and contain substantially no functional group other than the hydroxyl groups. Examples of the polyether polyol may include polyoxyalkylene polyol such as polyethylene glycol, polypropylene glycol, polytetramethylene glycol, polybutylene glycol and the like. Further, a homopolymer and a copolymer of the polyoxyalkylene polyols or mixtures thereof may also be employed. Particularly preferable copolymers of the polyoxyalkylene polyols may include an adduct of at least one compound selected from the group consisting of ethylene glycol, propylene glycol, diethylene glycol, dipropylene glycol, triethylene glycol, 2-ethylhexanediol-1,3,glycerin, 1,2,6-hexane triol, trimethylol propane, trimethylol ethane, tris(hydroxyphenyl)propane, triethanolamine, triisopropanolamine; with at least one compound selected from the group consisting of ethylene oxide, propylene oxide and butylene oxide. While such compounds are commercially available, methods for synthesizing such compounds are well known in the art. Non-limiting examples of commercially available polyols which may be used in the practice of the invention include polyethers such as polyether triol, such as those with a molecular weight of about 3000 to 9000, for example 4000-8000, for example about 6000, OH-terminated polybutadienes, such as those with a molecular weight of about 2000 to 4000, for example 25000 to 3500, such as about 2800, castor oil, and the OH-terminated prepolymer available under the tradename Loctite UK 8201 HF (Henkel).

Suitable further polyester polyols are formed from the condensation of one or more polyhydric alcohols having from about 2 to about 15 carbon atoms with one or more polycarboxylic acids having from about 2 to about 14 carbon atoms. Examples of suitable polyhydric alcohols include ethylene glycol, propylene glycol such as 1,2-propylene glycol and 1,3-propylene glycol, glycerol, pentaerythritol, trimethylolpropane, 1,4,6-octanetriol, 1.4-butanediol, 1.3-butanediol, 1.5-pentanediol, 1.4-pentanediol, hexanediol, dodecanediol, octanediol, chloropentanediol, glycerol monallyl ether, glycerol monoethyl ether, diethylene glycol, 2-ethylhexanediol, 1,4-cyclohexanediol, 1,2,6-hexanetriol, 1,3,5-hexanetriol, 1,3-bis-(2-hydroxyethoxy)propane and the like. While such compounds are commercially available, methods for synthesizing such compounds are well known in the art. Commercially available semicrystalline polyester polyols useful in the invention include, for example, Dynacoll 7380, and 7360 (Creanova), Fomrez 66-32 (Crompton) and Rucoflex S-105-30 (Bayer).

Suitable hydroxyl polycarbonates can be obtained by reaction of carbon acid derivatives, e.g. diphenyl carbonate, dimethyl carbonate or phosgene with diols. Suitable examples of such diols include ethylene glycol, 1,2- and 1,3-propanediol, 1,3- and 1,4-butanediol, 1,6-hexanediol, 1,8-octanediol, neopentyl glycol, 1,4-bishydroxymethyl cyclohexane, 2-methyl-1,3-pro-panediol, 2,2,4-trimethyl pentanediol-1,3, dipropylene glycol, polypropylene glycols, dibutylene glycol, polybutylene glycols, bisphenol A, tetrabromobisphenol A as well as lactone-modified diols. The diol component preferably contains 40 to 100 wt. % hexanediol, preferably 1,6-hexanediol and/or hexanediol derivatives. More preferably, the diol component includes examples that in addition to terminal OH groups display ether or ester groups. The hydroxyl polycarbonates should be substantially linear. However, they can optionally be slightly branched by the incorporation of polyfunctional components, in particular low-molecular polyols. Suitable examples include glycerol, trimethylol propane, hexanetriol-1,2,6, butanetriol-1,2,4, trimethylol propane, pentaerythritol, quinitol, mannitol, and sorbitol, methyl glycoside, 1,3,4,6-dianhydrohexites. Suitable polycarbonate polyols are, without limitation, those obtainable under the trademark names Desmophen^{®} C3200 (Bayer) and Kuraray^{®} C2050 (Poly-(3-methyl-1,5-pentanediol, 1,6-hexanediol)carbonate; Kuraray).

The polyurethanes may be produced using commonly used polyisocyanates. Organic polyisocyanates, which may be used to practice the invention, include alkylene diisocyanates, cycloalkylene diisocyanates, aromatic diisocyanates and aliphatic-aromatic diisocyanates. Specific examples of suitable isocyanate-containing compounds include, but are not limited to, ethylene diisocyanate, ethylidene diisocyanate, propylene diisocyanate, butylene diisocyanate, trimethylene diisocyanate, hexamethylene diisocyanate, toluene diisocyanate, cyclopentylene-1,3-diisocyanate, cyclo-hexylene-1,4-diisocyanate, cyclohexylene-1,2-diisocyanate, 4,4'-diphenylmethane diisocyanate, 2,2-diphenylpropane-4,4'-diisocyanate, xylylene diisocyanate, 1,4-naphthylene diisocyanate, 1,5-naphthylene diisocyanate, m-phenylene diisocyanate, p-phenylene diisocyanate, diphenyl-4,4'-diisocyanate, azobenzene-4,4'-diisocyanate, diphenylsulphone-4,4'-diisocyanate, 2,4-tolylene diisocyanate, dichlorohexa-methylene diisocyanate, furfurylidene diisocyanate, 1-chlorobenzene-2,4-diisocyanate, 4,4',4"-triisocyanatotriphenylmethane, 1,3,5-triisocyanato-benzene, 2,4,6-triisocyanatotoluene, 4,4'-dimethyldiphenyl-methane-2,2',5,5-tetratetraisocyanate, and the like. While such compounds are commercially available, methods for synthesizing such compounds are well known in the art. Preferred isocyanate-containing compounds are those that are (crystalline) solids at room temperature, including but not limited to methylene diphenyl diisocyanate (MDI) and toluene diisocyanate (TDI) polymers, such as dimers or trimers, in particular TDI dimers with urea or urethdione bridges. In various embodiments, the polyisocyanate used is, without limitation, 4,4'-, 2,2'- or 2,4'-MDI.

The polyurethane may be obtained by using the polyisocyanate in molar excess with respect to the NCO to OH ratio. In such embodiments, the obtained polyurethane may be an NCO-terminated polyurethane. For this, the molar ratio of the NCO groups of said polyisocyanate(s) to the sum of hydroxyl groups of said polyol(s) is greater than 1.00:1.00, for example 1.1:1 but can range up to, for example 3:1 or 2:1. In some embodiments, the ratio of hydroxyl groups to NCO groups can be in the range having any combination of an upper limit selected from less than 0.99: 1, 0.975: 1, 0.95:1, 0.9:1, 0.85:1, 0.8:1, 0.75:1 or 0.7:1 and a lower limit selected from 0.65:1, 0.7:1, 0.75:1, 0.8:1, 0.85:1, 0.9:1. In some embodiments the ratio is in the range of 0.65 to 0.95:1. In specific embodiments the ratio is 0.7 to 0.9:1.

Alternatively, the NCO to hydroxyl group ratio may be less than 1:1, for example up to 1:3 or up to 1:2. The ratio may, in various embodiments, be in a range having any combination of an upper limit selected from less than 0.99: 1, 0.975: 1, 0.95:1, 0.9:1, 0.85:1, 0.8:1, 0.75:1 or 0.7:1 and having a lower limit selected from 0.65:1, 0.7:1, 0.75:1, 0.8:1, 0.85:1, 0.9:1. In some embodiments the NCO to hydroxyl ratio is in the range of 0.65 to 0.95:1. The excess of hydroxyl groups means that the PU obtained after curing is preferably hydroxyl-terminated.

These polyurethanes of the invention may be part of adhesive or coating compositions that also form part of the invention. These may additionally comprise all additives that are known and conventional for such adhesive and coating compositions.

Such additives inert to the other components comprised in the PU compositions of the present invention and conventionally used in the art of PU formulations to satisfy different properties and meet specific application requirements can optionally be included from 0 wt.-% to about 40 wt.-%, for example up to about 20 wt.-%, in the PU compositions. Such additives include, for example, diluents, plasticizers, fillers, drying agents, rheology modifiers, pigments, dyestuffs, curing catalysts, adhesion promoters, which may be incorporated in minor or larger amounts into the adhesive formulation, depending on the purpose.

In various embodiments, the composition is an adhesive composition, such as a reactive hot melt adhesive composition.

### EXAMPLES

### Methods

**Catalyst Testing for the Hydrogenation of the Aqueous 5-HMF Solution.** In a typical screening of catalysts and reaction conditions, oven dried 4 mL glass vials equipped with magnetic stirring bars were used. Each vial was charged with a specific amount of heterogenous catalyst in order to have a final metal content of 1.26 mg, corresponding to 1 wt% with respect to the used 5-HMF. Subsequently, 560 µL (1 mmol) of the crude aqueous 5-HMF solution (c = 1.79 mol/L) and 1-2 mL of ethanol were added into each vial. The vials were placed in an aluminum inlet and closed with PTFE/rubber septa pierced with a needle. Three vials were placed in the same aluminum inlet which was then transferred into a 300 mL stainless steel autoclave from Parr. The latter was purged three times with 20 bar of N₂ and afterwards, two times with 10 bar of H₂. Finally, the autoclave was pressurized with either 25 bar or 90 bar of H₂ and it was put into a pre-heated aluminum block at 100 °C. After the desired amount of time, the reactor was brought at room temperature and carefully depressurized. The reaction mixture was filtered over a short pad of celite, by using ethanol as the eluent. Solvents were evaporated from the collected solution; 1,4-dinitrobenzene was added as an internal standard and conversion and yields as well as *cis*/*trans* ratios were determined by NMR spectroscopy.

**Screening of Catalysts and Reaction Conditions for Borrowing Hydrogen.** In a typical reaction, an oven dried 10 mL pressure Schlenk tube from FengTecEx (product number: F580810) equipped with a magnetic stirring bar was used. The Schlenk tube was charged under argon atmosphere with the respective amount of catalyst and base inside the glovebox; it was sealed with a Teflon screw cap and it was taken out of the glovebox. Subsequently, the desired solvent (4.0 mL or 2.0 mL) and 1.0 mmol (132 mg) of degassed *cis*-enriched DHMTHF (92%/8% *cis*:*trans*) were added under a constant argon flow. The Schlenk tube was again sealed with a Teflon screw cap and put into a pre-heated oil bath at the desired temperature in which the mixture was stirred (500 rpm) for 24 hours. Afterwards, the reaction was stopped and allowed to cool at room temperature. The mixture was filtered over a short pad of silica under a constant argon flow by using diethyl ether as the eluent. The solvents were then removed on the rotary evaporator and the yield of DHMTHF was determined. Finally, an aliquot of the obtained product was analyzed *via* NMR-spectroscopy to determine the *cis*/*trans* ratio.

**Acetylation of *cis*/*trans-*DHMTHF.** 20.0 g (151 mmol) of DHMTHF with a content of 44% of the *trans* isomer and acetic anhydride (34.0 mL, 360 mmol) were added into a round bottom flask equipped with a reflux condenser. The mixture was heated up to 140 °C under stirring, and after reaching this temperature, stirring was continued for further 3 h. Afterwards, the mixture was allowed to cool down and the formed acetic acid was removed under high vacuum at 70 °C. The obtained brownish liquid was the corresponding diacetyl ester of DHMTHF and it was used without any further purification for the following steps. Yield: 32.3 g (99%).

**Crystallization of *trans*-DHMTHF.** 245.0 g of diacetyl-DHMTHF (61% *cis*/*39% trans*) were transferred into a round bottom flask and dissolved in 80 mL of dry Et₂O. The *trans*-enriched diacetyl-DHMTHF precipitated out of the solution as a white crystalline solid after the crude mixture was kept at -15 °C for more than 12 h. The crystalline precipitate was filtered over a Büchner funnel with filter paper (both were pre-chilled in the refrigerator) and washed three times with cold Et₂O. Finally, the crystalline solid was dried under high vacuum. Yield: 63.0 g (66%), *cis*/*trans* ratio: 15%/85%.

**Hydrolysis of Diacetyl-DHMTHF Esters (using the Example of the trans-enriched Diacetyl-DHMTHF).** A 1000 mL two-necked round bottom flask equipped with a condenser was charged with a magnetic stirring bar and 63.0 g of *trans*-enriched diacetyl-DHMTHF (291 mmol). 40.0 mL of ethanol were added to pre-dissolve the crystalline material. Subsequently, 600 mL of a NaOH 4 M aqueous solution were added dropwise over 2 h under stirring at 40 °C. After complete addition, the mixture was stirred for additional 48 h. Afterwards, water was removed on the rotary evaporator and the obtained viscous suspension was filtered over silica by using EtOAc as the eluent. After removing the volatiles under reduced pressure, the *trans*-enriched DHMTHF was obtained as a colourless liquid by vacuum (0.3 mbar) distillation at 130 °C. Yield: 27.6 g (72%), *cis*/*trans* ratio: 15%/85%.

**Synthesis of Polyesters-Polyols.** A 250 mL three-necked flask equipped with a mechanical stirrer, a Vigreux column and a distillation bridge attached to another 250 mL round bottom flask for collecting water, was charged with dodecanedioic acid and DHMTHF. The entire apparatus was purged for 1 h with nitrogen before the mixture was heated up to 120 °C. At this temperature, the reaction was kept for 1 h and then the temperature was further increased to 140 °C. Afterwards, the temperature was continuously increased by 20 °C up to 220 °C and after each increase, it was hold for at least 1 h. At 220 °C, vacuum was applied, slowly going from 850 mbar to 15 mbar. After 27 h at 220 °C and 15 mbar, the reaction mixture was cooled down at 120 °C and the vacuum was released. Titanium (IV) isopropoxide was added as a catalyst, the mixture was heated up again to 220 °C and vacuum (15 mbar) was applied. Finally, after additional 7 h at 220 °C under vacuum, the reaction mixture was cooled down, and the successful conversion was evaluated *via* acid number titration with a 0.1 M KOH aqueous solution and phenolphthalein as indicator.

**Formation of Polyurethane Films.** A 250 mL three-necked flask equipped with a mechanical stirrer and a condenser was charged with 50 g of polyester (example: OH-number 27). The polymer was heated up to 80 °C and kept at this temperature under vacuum (<0.001 mbar) for 1.5 hours. Afterwards, 2.2 equivalents of 4,4'-MDI (6.64 g) were added, and the mixture was stirred for 1 h under nitrogen atmosphere. The obtained polymers were used to cast films of 1 mm thickness which were stored for one week at ambient conditions.

### Example 1: Hydrogenation of aqueous 5-HMF solution

56.0 mL (100 mmol) of the aqueous 5-HMF solution (c = 1.79 mol/L) and 140.0 mL of ethanol were added into a 300 mL stainless steel autoclave (Parr) equipped with a PTFE cross-stirring bar (length: 38 mm). Subsequently, Raney-Nickel catalyst (2.5 g solid catalyst) in form of an aqueous suspension was added to the mixture. Afterwards, the autoclave was closed, purged three times with 20 bar of N₂, two times with 20 bar of H₂ and finally pressurized with 90 bar of H₂ and placed into an aluminum block pre-heated at 100 °C. The mixture was stirred for 12 h at 650 rpm, and after that the autoclave was cooled to room temperature, repressurized with 90 bar of H₂, and placed again into the aluminum block for other 12 h at 100 °C. After a total of 24 h, the reactor was carefully depressurized at room temperature. The reaction mixture was filtered over a pleated filter, by taking special care of continuously rinsing the filter paper with ethanol. After evaporation of the solvents, a brownish liquid was obtained. DHMTHF was then collected by distillation under vacuum (0.4 mbar) at 130 °C as a colorless viscous liquid in 92% yield (12.1 g) with a *cis*-content of 92%.

**Table 1. Catalyst testing for the hydrogenation of aqueous 5-HMF.**

| | | | | | |
|---|---|---|---|---|---|
| 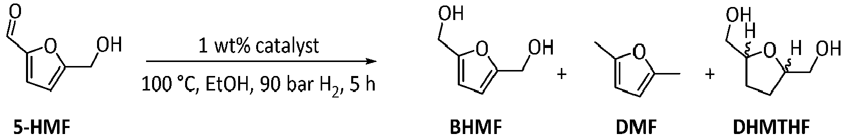 | | | | | |

| Entry | Catalyst | Conv. 5-HMF^{a} | Y BHMF^{a} | Y DMF^{a} | Y DHMTHF^{a, b} |
|---|---|---|---|---|---|
| 1 | **Ra-Ni^{c}** | 86% | 7% | 0% | 78% (92%/8%) |
| 2 | **Ru/C** | >99% | 2% | 1% | 96% (85%/15%) |
| 3 | **Pd/Al** | 92% | 16% | 0% | 74% (88%/12%) |
| 4 | **Pd/C** | 99% | 1% | 1% | 92% (80%/20%) |
| 5 | **Pt/C** | 80% | 75% | 4% | <1% (n.d.) |

| | | | | | |
|---|---|---|---|---|---|
| Reaction conditions: 0.56 mL (1.0 mmol) aqueous 5-HMF solution (c = 1.79 mol/L), 2.0 mL EtOH, 1 wt% (based on metal) heterogenous catalyst, 100 °C, 90 bar H₂, 5 h; a - determined *via* NMR using 1,4-dinitrobenzene as internal standard (added after hydrogenation); b - in brackets, the ratios between the *cis* and the *trans* isomers are reported; c - 20 mg Ra-Ni slurry in water was used. Conv. = conversion; Y = yield | | | | | |

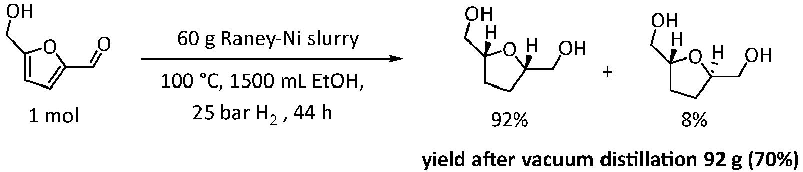

### Example 2: Isomerization of cis- to trans-DHMTHF through Borrowing Hydrogen Methodology

A 300 mL autoclave (Parr) was charged with a PTFE cross-stirring bar and 23.8 g of *cis*-enriched DHMTHF-diol (180 mmol). Afterwards, the autoclave was closed, and vacuum was applied for 30 min. Subsequently, 263.9 mg of **Ru-3** (0.25 mol%) which was weighted in a vial inside the glovebox, was added into the autoclave under a constant argon flow. Finally, 180.0 mL of toluene and 11.7 mL of KO*^{t}*Pen (10 mol%; 1.7 mol/L toluene solution) were added under argon. The autoclave was purged three times with 20 bar of N₂ and it was put into an aluminum block pre-heated at 100 °C, where the mixture was stirred (600 rpm) for 16 h. Afterwards, the reaction mixture was allowed to cool at room temperature and it was transferred into a round-bottom flask. Toluene was removed on the rotary evaporator and the final mixture was filtered through silica under a constant argon flow by using diethyl ether as the eluent. Diethyl ether was then removed on the rotary evaporator and the DHMTHF *cis*/*trans*-mixture was obtained as a viscous and colorless liquid in 94% yield (22.3 g) by distillation under vacuum at 0.3 mbar and 130 °C.

**Table 2. Catalyst screening for the isomerization of cis-enriched DHMTHF.**

| | | | | |
|---|---|---|---|---|
| 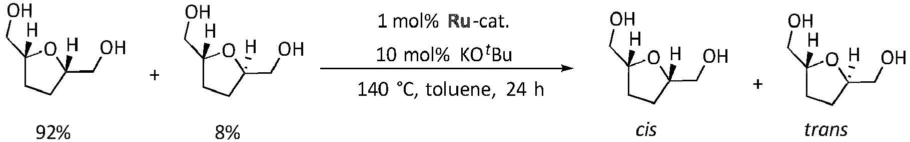 | | | | |

| Entry | Catalyst | Y DHMTHF^{a} | *Cis* Content^{b} | *Trans* Content^{b} |
|---|---|---|---|---|
| 1 | **Ru-1** | 90% | 74% | 26% |
| 2 | **Ru-2** | 87% | 56% | 44% |
| 3 | **Ru-3** | 89% | 70% | 30% |
| 4 | **Ru-4** | 93% | 87% | 13% |
| 5 | **Ru-5** | 91% | 57% | 43% |
| 6 | **Ru-6** | 91% | 75% | 25% |
| 7 | **Ru-7** | 85% | 67% | 33% |
| 8 | **Ru-8** | 95% | 57% | 43% |
| 9 | **Ru-9** | 93% | 76% | 24% |

| | | | | |
|---|---|---|---|---|
| Reaction conditions: 1 mmol of ***cis*-enriched** DHMTHF, 4 mL toluene, 140 °C, 24 h; a - isolated yield; b - *cis*/*trans* ratio determined by NMR-spectroscopy. | | | | |

**Table 3. Optimization of the reaction conditions for the isomerization of cis-enriched DHMTHF.**

| | | | | | | |
|---|---|---|---|---|---|---|
| 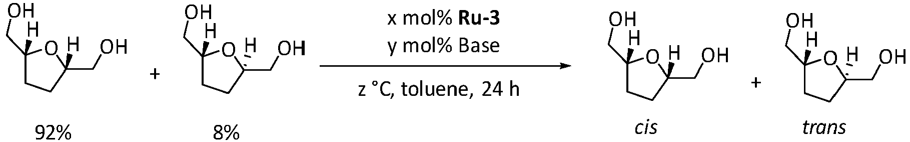 | | | | | | |

| Entry | Catalyst (mol%) | Base (mol%) | T [°C] | Y^{a} DHMTHF | *Cis* Content^{b} | *Trans* Content^{b} |
|---|---|---|---|---|---|---|
| 1 | **Ru-2** (0.5) | KOtBu (10) | 100 | 91% | 55% | 45% |
| 2 | **Ru-3** (1.0) | KOtBu (10) | 100 | 90% | 56% | 44% |
| 3 | **Ru-3** (1.0) | KO^{t}Bu (10) | 60 | 93% | 92% | 8% |
| 4 | **Ru-3** (1.0) | KO^{t}Bu (10) | r.t. | 95% | 92% | 8% |
| 5 | **Ru-3** (1.0) | KO^{t}Bu (5) | 100 | 93% | 88% | 12% |
| 6 | **Ru-3** (1.0) | KO^{t}Bu (20) | 100 | 88% | 56% | 44% |
| 7 | **Ru-3** (1.0) | - | 100 | 92% | 92% | 8% |
| 8 | **-** | KO^{t}Bu (10) | 100 | 84% | 92% | 8% |
| 9^{c} | **Ru-3** (1.0) | KO^{t}Bu (10) | 100 | 88% | 76% | 24% |
| 10 | **Ru-3** (1.0) | KO^{t}Pen (10) | 100 | 94% | 55% | 45% |
| 11 | **Ru-3** (0.25) | KO^{t}Pen (10) | 100 | 90% | 55% | 45% |
| 12 | **Ru-3** (0.1) | KO^{t}Pen (10) | 100 | 92% | 57% | 43% |

| | | | | | | |
|---|---|---|---|---|---|---|
| Reaction conditions: 1 mmol of *cis*-enriched DHMTHF, 2 mL solvent, 24 h; a - isolated yield; b - *cis*/*trans* ratio determined by NMR-spectroscopy; c - reaction was performed in a 4 mL vial with a PTFE/rubber septum pierced with a needle inside a 300 mL autoclave under 3 bar of H₂ | | | | | | |

### Example 3: Influence of the DHMTHF diastereomers on the properties of polyesters and polyurethanes

In order to examine the impact of the incorporation of the two diastereomers of DHMTHF in polymers for adhesives, the previously prepared diastereomeric mixtures (92:8, 57:43, 15:85) were used in combination with dodecanedioic acid (DDA) for the synthesis of novel and potentially 100% bio-based polyester polyols. Polyesters were prepared accordingly to a two-stage melt condensation process in which (I) the pre-condensation at ambient pressure in concomitant with a gradual temperature increase from 120 °C to 220 °C and (II) the condensation in the presence of catalytic amounts of titanium(IV)isopropoxide under vacuum were performed. The found hydroxyl numbers for the synthesized polyesters were in the desired range (OH-N = 25-34 mg g⁻¹), and only minor amounts of side products are formedThus, DHMTHF was successfully employed as a monomer in the industrially preferred melt condensation process yielding polyester polyols with low acid numbers (A-N = 1-3 mg g⁻¹) in concomitant with high viscosities at room temperature and honey-like viscosities at 80 °C (see Table **4**).

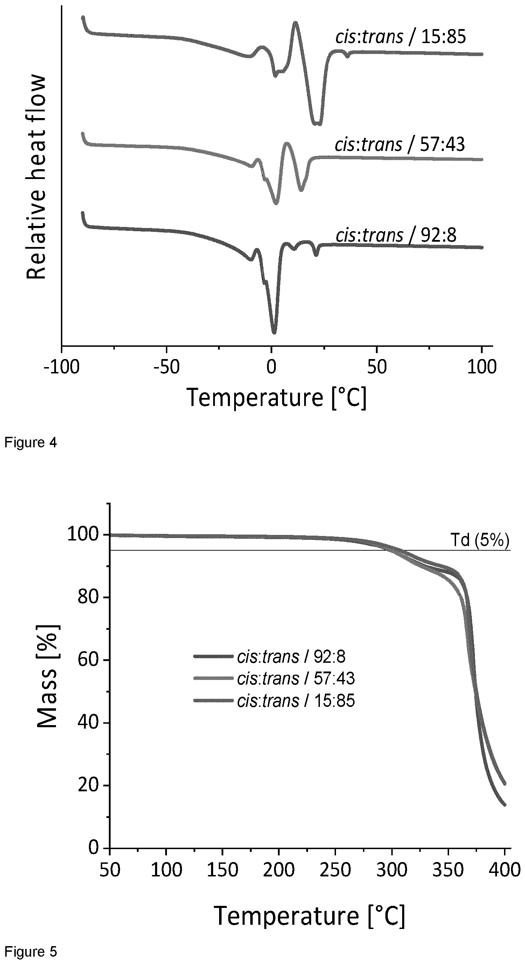

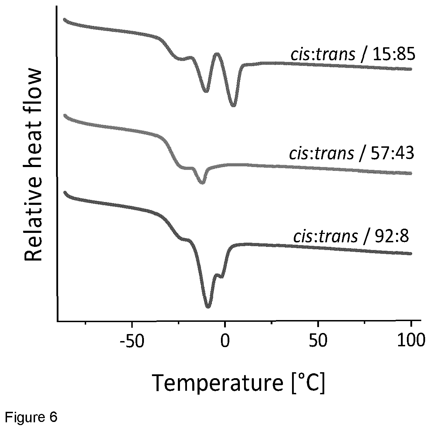

To study the influence of the two different diastereomers on the thermal behavior of the derived polymers, differential scanning calorimetry (DSC) was performed (Figure 4). Interestingly, independently from the used diastereomeric mixture, all synthesized polyester polyols showed semicrystalline properties indicated by the presence of at least two melting points. Notably, the integration areas of the latter resulted to be proportional to the molar fractions of the *cis-* and *trans*-DHMTHF within the diol mixture. Particularly, the polyester polyol obtained from the *cis* enriched DHMTHF exhibited one main crystalline domain with a peak temperature at 1 °C which is likely related to the *cis* isomer, and two small melting peaks centered at 11 °C and 21 °C, which can be the result of crystalline phases with a mixed and a *trans* enriched content, respectively (Figure 4, bottom curve). Accordingly, in the *trans* enriched DHMTHF based polymer, the major crystalline phase was found to be the one with a melting peak at 21 °C (Figure 4, top curve).

Remarkably, the polymer derived from the 57:43 *cis*/*trans* DHMTHF mixture also showed two distinctive melting points (Figure 4, middle curve). Interestingly, the higher was the excess of *trans*-DHMTHF used in the synthesized polyesters, the higher resulted to be the degree of crystallinity of the latter. Notably, the crystallization temperatures (T_{c}) for all three polyester polyols were almost identical and, in each case, just one peak was observed (data not shown).

To use the synthesized polyester polyols for the preparation of polyurethane pre-polymers for moisture curing, 4,4'diphenylmethane diisocyanate (4,4'-MDI) was reacted in a slight excess with the polyester polyols in order to produce isocyanate-terminated polymers that were afterwards post-cured in a climate chamber (23 °C, 50% relative humidity) to afford poly(urethane-urea) polymers (PU's). Thermogravimetric analysis of the latter revealed good thermal stability in all cases with a 5% mass loss at around 300 °C (see Figure 5).

Moreover, the mechanical properties of the obtained polymers were investigated by performing stress strain tests. As summarized in Table 5, the PU film obtained from the *cis*/*trans* DHMTHF polyester polyol exhibited good tensile strength and, at the same time, a remarkably high elongation. This combination of properties is highly desired for many applications, like for example to join materials with different coefficients of thermal expansion, or for gluing windows and other components in car interiors. PU's derived from *cis-* and *trans*-enriched DHMTHF polyester polyols showed also acceptable values for both tensile strength and elongation at break, albeit lower than those obtained with the *cis*/*trans* DHMTHF polyester polyol. Hence, it can be concluded that a balanced ratio of *cis*/*trans* DHMTHF into polyester polyols improves the mechanical performance.

**Table 4. Results of the polycondensation of DHMTHF with 1,12-dodecanedioic acid.**

| | | | | | | | | | | |
|---|---|---|---|---|---|---|---|---|---|---|
| 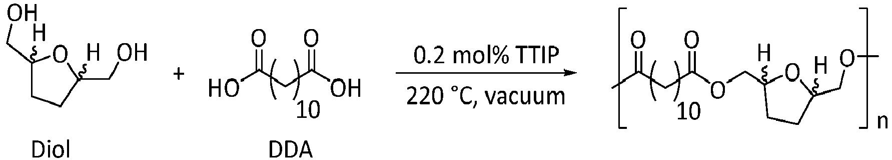 | | | | | | | | | | |

| Entry | *Cis*/*Trans* | OH-N^{a} [mg/g] | A-N^{a} [mg/g] | Mₙ(Calc.)^{b} [g/mol] | Mₙ(OH-N)^{a} [g/mol] | Mₙ(GPC) [g/mol] | *Ð^{c}* | *η*[Pas]^{d} | Tₘ[°C] | T_{c}[°C] |
|---|---|---|---|---|---|---|---|---|---|---|
| 1 | 92/8 | 27 | 3 | 3741 | 4156 | 5022 | 3.1 | 122 (4.2) | 1/11/21 | -14 |
| 2 | 57/43 | 34 | 1 | 3741 | 3301 | 4175 | 2.7 | 68 (2.4) | 2/14 | -15 |
| 3 | 15/85 | 25 | 1 | 3741 | 4489 | 6574 | 2.3 | n.d.^{e} (5.4) | 5/21 | -13 |

| | | | | | | | | | | |
|---|---|---|---|---|---|---|---|---|---|---|
| a-determined by titration with KOH_{aq} (0.1 mol/L); OH-N analogous to DIN 53240-1; b - targeted molecular weight; c - measured by GPC; d - viscosity at 23 °C, in brackets viscosity at 80 °C; e - polyester polyol was a solid at r.t. | | | | | | | | | | |

**Table 5. Thermal and mechanical properties of the corresponding PU films.^{a}**

| Entry | *Cis*/*Trans^{b}* | ε_{break} [%] | *F*_{break} [Nmm^{- 2}] | T_{deg} (5%)^{c} [°C] | Tₘ^{d}[°C] | T_{g}^{d} [°C] |
|---|---|---|---|---|---|---|
| 1 | 92/8 | 630 | 2.0 | 299 | -9/-1.5 | -33 |
| 2 | 57/43 | 1055 | 12.2 | 300 | -12 | -32 |
| 3 | 15/85 | 497 | 3.0 | 307 | -10/5 | -33 |

| | | | | | | |
|---|---|---|---|---|---|---|
| a - DIN 53504-SF3A-bones from 4,4'-MDI chain extended films; ε_{break}: elongation at break; *F*_{break}: stress at break; b - diastereomeric DHMTHF ratio used for the synthesis of the corresponding polyester polyols; c- measured by TGA; d - determined by DSC. | | | | | | |

In summary, the synthesis of different *cis*/*trans* DHMTHF mixtures from an aqueous 5-HMF solution was demonstrated on a multigram scale. Particularly, Raney-Nickel was proven to be active in the direct hydrogenation of 5-HMF towards ***cis*-enriched** DHMTHF not only with pure 5-HMF as the starting material, but also in the presence of considerable high amounts of impurities. Furthermore, *cis*-DHMTHF was herein tested for the first time in a borrowing hydrogen reaction which allowed a significant increase of the *trans* isomer content in concomitance with high isolated yields. Remarkably, commercially available Ru-complexes were found to efficiently catalyze the isomerization of *cis* to *trans*-DHMTHF under basic conditions at 100 °C. Interestingly, the acetylation of the *cis*/*trans* mixture allowed the separation of the *trans* isomer *via* crystallization in, for example diethyl ether, at -15 °C. Additionally, it was shown that the remaining liquid containing the ***cis*-enriched** DHMTHF diacetate, could after hydrolysis undergo once more the isomerization/crystallization sequence qualifying the latter as a suitable cascade for the synthesis of *trans*-DHMTHF. Finally, novel polyester polyols were synthesized from different DHMTHF mixtures and diacid, which is preferably also available from renewable sources. DSC analysis revealed that the higher the amount of *trans*-DHMTHF the higher the degree of crystallinity of the resulting polymer.

## Claims

1. Method for the production of di(hydroxymethyl)tetrahydrofuran (DHMTHF) from 5-hydroxymethylfurfural (5-HMF), the method comprising hydrogenation of a solution of 5-HMF, optionally an aqueous solution, in the presence of a heterogeneous catalyst, wherein the 5-HMF is crude 5-HMF.

2. The method of claim 1, wherein the 5-HMF is
(a) 5-HMF obtainable as a side stream product in a hydrothermal carbonization process, optionally of sugars and/or lignocellulosic material; and/or
(b) 5-HMF having a purity of less than 95% .

3. The method of claim 1 or 2, wherein the heterogeneous catalyst is a syn facial hydrogenation mode catalyst, preferably selected from Raney nickel, Ru/C (ruthenium on carbon), Pd/AI (palladium onalumina), Pd/C (palladium on carbon), Pd/Si (palladium on silica), Ru/AI (ruthenium onalumina), Raney cobalt, Raney copper, more preferably Raney nickel.

4. The method of any one of claims 1 to 3, wherein the hydrogenation reaction is carried out
(a) in the presence of ethanol in an amount of at least 1.3 mL ethanol per 1 mmol 5-HMF, preferably 1.4 to 3.0 mL ethanol per 1 mmol 5-HMF;
(b) under a H₂ pressure of at least 10 or at least 20 bar, preferably at least 50 bar, more preferably 80 to 100 bar;
(c) at a temperature of 80 to 120°C, preferably at about 100°C; and/or
(d) at a reaction time of at least 5 hours, preferably 5 to 60 hours.

5. Method for the enrichment of trans-DHMTHF from a mixture of cis-/trans-DHMTHF, the method comprising
(A) reacting a mixture of cis-/trans-DHMTHF at elevated temperatures in the presence of a suitable metal catalyst for a metal catalyzed borrowing hydrogen reaction, a base and a suitable solvent; or
(B)
(i) acetylating the mixture of cis- and trans-DHMTHF, optionally with acetic anhydride, optionally at elevated temperatures, to obtain a mixture of diacetyl esters of cis- and trans-DHMTHF;
(ii) crystallizing the diacetyl ester of trans-DHMTHF from a solution of the mixture of diacetyl esters of cis- and trans-DHMTHF in a suitable solvent, at a temperature that allows preferential crystallization of trans DHMTHF;
(iii) isolating the crystallized diacetyl ester of trans-DHMTHF; and
(iv)hydrolyzing the diacetyl ester of trans-DHMTHF under basic conditions to obtain enriched trans-DHMTHF.

6. The method of claim 5 (A), wherein
(a) the mixture of cis-/trans-DHMTHF has a cis to trans ratio of at least 1, preferably at least 2, more preferably 3-20;
(b) the catalyst is a ruthenium, cobalt, manganese or iron complex catalyst, preferably a ruthenium complex bearing tridentate pincer ligands, more preferably a ruthenium complex selected from Ru-2, Ru-3, Ru-5 and Ru-8;
(c) the reaction temperature is 80°C or more, preferably 80-120°C;
(d) the base is a strong base, preferably selected from metal alkoxides, more preferably selected from potassium tert butoxide, sodium tert butoxide, potassium tert pentoxide, sodium tert pentoxide, sodium methoxide, potassium methoxide, sodium ethoxide, potassium ethoxide;
(e) the solvent is selected from the group consisting of heptane, THF, 1,4-dioxane, toluene, sulfolane, acetonitrile, 2-MeTHF, and mixtures thereof;
(f) at a reaction time of 2 to 48 hours, preferably about 4 to 24 hours;
(g) the base is used in amounts of at least 7 mol.-%, preferably at least 10 mol-%;
(h) the catalyst is used in amounts of 1 mol.-% or less, preferably about 0.5 mol.-% or less;
(i) the reaction is not carried out under hydrogen atmosphere;
(j) the content of the trans diastereomer in the trans enriched product is at least 10 mol-% higher than in the mixture of cis-/trans-DHMTHF used as a starting material, preferably at least 20 mol-% higher.

7. The method of claim 5(B), wherein
(1) the mixture of cis-/trans-DHMTHF has a cis to trans ratio of 3 or less, preferably 2.5 or less, more preferably 2 or less, most preferably 1.5 or less; and/or
(2) the cis-DHMTHF enriched solution that remains after step (iv) is hydrolyzed under basic conditions and first subjected to the method according to claim 5(A) and the resulting mixture of cis- and trans-DHMTHF is subjected again to the method according to claim 5(B), wherein this sequence of steps is, optionally, repeated multiple times to achieve a nearly stereoselective formation of trans-DHMTHF.

8. Polyester polyol
(A) obtainable by reacting a diastereomeric mixture of cis-/trans-DHMTHF with a suitable diacid, wherein the diastereomeric mixture of cis-/trans-DHMTHF has a cis- to trans-DHMTHF ratio of about 70:30 or less; or
(B) comprising monomeric units of formula (la) and (lb) 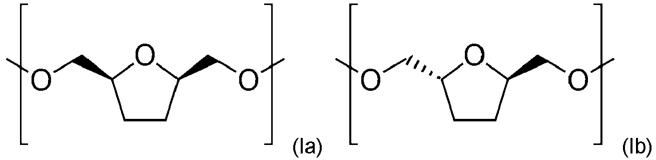
in a molar ratio of (la) to (lb) of 70:30 or less, preferably 70:30 to 30:70, wherein the monomeric units of formulae (la) and (lb) preferably constitute at least 5 mol-%, preferably at least 20 mol-%, more preferably at least 50 mol-% of the total polyol units in said polyester polyol.

9. Method for the production of a polyester polyol, the method comprising reacting a diastereomeric mixture of cis-/trans-DHMTHF with a suitable diacid.

10. The polyester polyol of claim 8 or the method of claim 9, wherein
(a) the diacid is a dicarboxylic acid, preferably an aromatic or aliphatic dicarboxylic acid with 4 to 30 carbon atoms;
(b) the diastereomeric mixture of cis-/trans-DHMTHF has a cis- to trans-DHMTHF ratio of about 30:70 to 70:30, preferably about 40:60 to about 60:40;
(c) the diastereomeric mixture of cis-/trans-DHMTHF is obtained according to any one of the methods of claims 1-7;
(d) the reaction is carried out using a two-stage melt condensation process comprising a precondensation step at ambient pressure with a gradual temperature increase of from about 120 to about 220°C and a subsequent condensation step under reduced pressure; and/or
(e) the polyester polyol has a hydroxyl number of 10 to 150 mg KOH/g.

11. Polyurethane obtainable by reacting a polyester polyol according to claims 8 or 10 or obtainable according to the methods of claims 9 or 10 with a polyisocyanate.

12. Method for the production of a polyurethane comprising reacting a polyester polyol according to claims 8 or 10 or obtainable according to the methods of claims 9 or 10 with a polyisocyanate.

13. The polyurethane of claim 11 or the method of claim 12, wherein
(a) the polyisocyanate is used in molar excess with respect to the NCO to OH ratio; and/or
(b) the polyisocyanate is a diisocyanate, optionally 4,4'-MDI.

14. Composition comprising (a) the diastereomeric mixture of cis-/trans-DHMTHF as obtained according to any one of the methods of claims 1-7; (b) a diastereomeric mixture of cis-/trans-DHMTHF in a molar ratio of cis to trans diastereomer of 70:30 or less, preferably 70:30 to 30:70; (c) the polyester polyol according to claim 8 or obtainable according to the methods of claims 9-10; or (d) the polyurethane according to claim 11 or obtainable according to the method of claims 12-13.

15. The composition of claim 14, wherein the composition is an adhesive composition.
